# EUROPEAN PATENT APPLICATION

(11) **EP 4 112 107 A1**
(43) Date of publication of application: **04.01.2023**
(21) Application number: 22186390.5
(22) Date of filing: 22.07.2022
(51) Int. Cl.: A61M 5/32, A61M 5/24, A61M 5/315

(54) **SAFETY MECHANISM FOR A DRUG DELIVERY DEVICE**

(71) Applicant: Ypsomed AG, 3401 Burgdorf (CH)
(72) Inventor: Scheurer, Simon, 3006 Bern (CH); Hostettler, Patrick, 3415 Hasle (CH)
(74) Representative: Kleiner, Stefan

(57) **Abstract**

The invention relates to a safety mechanism for a drug delivery device (1) for dispensing a liquid drug from a reservoir (15) through a needle (18). The safety mechanism comprises a housing (80) defining a longitudinal axis, a needle cover (20) movable along the longitudinal axis between a needle covering position and a retracted position, a movable lock element (32) that can be in a locking position to prevent the needle cover (20) from being moved out of the needle covering position. The mechanism includes a trigger member (50) movable along the longitudinal axis relative to the housing (80) wherein the trigger member (50) is operatively coupled to the lock element (32) and a movement of the trigger member (50) along the longitudinal axis causes the lock element (32) to move out of the locking position allowing the needle cover (20) to be moved out of the needle covering position.

## Description

### FIELD OF THE INVENTION

The present invention relates to drug delivery devices or medicament delivery devices for injecting, delivering, administering, infusing or dispensing substances and/or liquids such as insulin, hormone preparations or vaccines. It departs from a safety mechanism for a drug delivery device comprising a needle cover and a lock element that can be in a locking position, in which the lock element engages the needle cover to prevent the needle cover from being moved out of a needle covering position.

### BACKGROUND OF THE INVENTION

A variety of diseases exist that require regular treatment by subcutaneous administration of a medicament, and a number of drug delivery devices have been developed to support a patient in accurately and controllably delivering an amount of drug in a self-administration process. Delivery devices include injection devices that are removed from the injection site after each medication event or drug delivery process, as well as infusion devices with a cannula or needle that remains in the skin of the patient for a prolonged period of time.

By way of example, diabetes may be treated by self-administration of insulin or its derivatives with the help of multi-variable-dose insulin injection pens. An injection pen device generally has an elongate device body defining a longitudinal main device axis. An automatic injection device has a motor or a drive spring for biasing a plunger rod and shifting a piston in a container barrel, wherein the drive spring may have to be charged or strained manually prior to injection of a dose. A manually powered delivery drive requires a user to manually provide the energy to move the piston, for instance by applying a distal force component to the injection device.

The medicament dose to be injected may be manually selected by turning a dosage knob and observing the actual dialed dose from a dose window or display of the insulin pen. A dose is dispensed by inserting the needle into a suited portion of human skin and by moving the piston manually or by pressing a release button of an automatic injection device. Automatic injection devices may comprise an electronic dose dial mechanism to automatically set a dose.

Also known is the use of autoinjectors with prefilled syringes. Autoinjectors usually comprise a body for housing the syringe as well as an automatic drive mechanism in order to move the plunger of the syringe upon actuation of the autoinjector. The drive mechanism typically comprises a source of drive, such as a motor or strong spring for moving a transfer element, for example a rod, which acts on the plunger of the syringe.

For safety and hygiene reasons it is desirable that the needle does not protrude from a housing of the autoinjector with the exception of the time when the needle is used for injection of a medicament. Thus, either the autoinjector moves the needle out of the housing for the injection and back into the housing after injection or the housing provides a needle cover or needle guard which may be moved to unsheathe the needle for injection and which may be moved back in a needle covering position after the injection.

The majority of autoinjectors are configured as single use devices which incorporate both the syringe and the drive mechanism in the same housing. Such devices are usually disposable for hygiene reasons.

Disposable autoinjectors comprising an electric actuator or an electronic control require a source of energy which is usually in the form of a battery. However, in this case, the autoinjectors should not be disposed in the regular waste, but have to be subjected to special disposal or to recycling, which is an additional burden to the patient. Further, disposing a battery, motor and/or electronics after a single use is a waste of resources and increases the costs of the autoinjector.

In order to account for a need to handle and dispose the autoinjector parts differently semi-reusable autoinjectors have been developed. Such autoinjectors typically comprise a reusable drive unit as well as a disposable syringe unit which may be releasably coupled or attached to the drive unit. The drive unit usually includes the drive mechanism and electronics whereas the syringe unit includes the syringe with the injection needle and a needle cover sleeve. The user can thus dispose of the syringe unit when empty or after use and can load the drive unit with a new syringe unit for an upcoming injection.

Different mechanism for covering and uncovering the injection needle are known in the art. Typically, a cover sleeve or movable sheath can be unlocked and subsequently retracted to expose the needle and prepare the delivery device for an injection.

WO12038721 A1 discloses a semi-reusable autoinjector with a syringe unit including a syringe and with a drive unit including a torsion spring and a gear to automatically dispense the drug from the syringe. The drive unit comprises an opening to accommodate the syringe unit. In a bottom plate in the opening two forwardly projecting lugs are designed to enter into recesses in the syringe unit housing upon insertion of the syringe unit to unlatch a rearward collar of a syringe shield. This allows the syringe shield to move to a retracted position.

WO 2010/046569 discloses a reusable autoinjector with a syringe unit that can be coupled to a drive unit by a bayonet connection. Once a cam of the drive unit is inserted in a slot in the syringe unit housing a cover sleeve is unlocked and can be moved to unsheathe the needle.

WO 2021/069106 discloses an autoinjector comprising a syringe unit and a drive unit. When an axially protruding locking pin of the drive unit is pushed through in a pin opening in the syringe unit, it comes in contact with a syringe holder arm. This results in a deflection of the holder arm, which in turn unlocks a needle cover sleeve and allows it to move in the proximal direction.

### DESCRIPTION OF THE INVENTION

It is an objective of the invention to provide a space-saving design and a safe and reliable release of a needle protection of a drug delivery device.

This objective is achieved by a safety mechanism for a drug delivery device or a method according to the independent claims. Preferred embodiments are evident from the dependent claims.

According to the invention a safety mechanism for a drug delivery device for dispensing a liquid drug from a reservoir through a needle is provided. The safety mechanism comprises
- a housing defining a longitudinal axis, the housing is preferably adapted to accommodate a dispensing mechanism for dispensing the drug and is adapted to accommodate at least partially the reservoir with the drug;
- a needle cover movable along the longitudinal axis between a needle covering position, in which the needle is covered by the needle cover and a retracted position, in which the needle cover is moved away from a tip of the needle and the needle is exposed and protrudes from the housing;
- a movable lock element that can be in a locking position, in which the lock element preferably engages the needle cover or a needle cover supporting member or the housing to prevent or block the needle cover from being moved out of the needle covering position towards a retracted position.

The safety mechanism further includes a trigger member (preferably exclusively) movable along the longitudinal axis relative to the housing. The trigger member is operatively and permanently coupled (direct or indirect via an intermediate member) to the lock element and can actuate the lock element such that a movement of the trigger member along the longitudinal axis causes the lock element to move out of the locking position. Thus, the needle cover is no longer blocked by the lock element and the needle cover can be moved out of the needle covering position towards a retracted position. The delivery device is then ready for an injection as the needle is exposed and accessible.

In other words, with an exclusive longitudinal movement of the trigger member (manually or automatically driven) the needle cover can be unlocked and retracted towards an uncovering position.

In contrast to known approaches that focus on a radial release trigger (in a plane perpendicular to the longitudinal axis) according to the invention the trigger member is movable along the longitudinal axis. As the trigger member does not move in radial direction the dimensions of the safety mechanism and thus the dimension of the drug delivery device in radial direction can be reduced compared to known mechanism. Thus, the arrangement according to the invention provides for a space-saving design. In view of WO12038721 the invention provides at least an alternative design with a trigger member movable relative to the housing.

The trigger member unlocks the locking element and thus the trigger member may also named as unlocking member. The trigger member may be, for example, a sleeve or a nut movable relative to the housing. The trigger member may be moved manually by a user force or automatically by an automatic drive such as an electric motor or a preloaded spring member. In a preferred embodiment the trigger member is sleeve-shaped and coaxially arranged to a reservoir or syringe inside the housing. This allows for a space-saving design.

Furthermore, the trigger member can actuate the lock element. That means the lock element can be brought into contact with the lock element either directly or indirectly via an intermediate member. In the latter case the trigger member contacts or engages the intermediate member which in turn contacts or engages the lock element. In this way, a movement of the trigger member is transferred to the lock element to move the lock element out of the locking position towards a release position in which the lock element does not hinder or block the needle cover and the needle cover is movable out of the covering position towards a retracted position.

Optionally, the longitudinal shifting or movement of the trigger member can be transferred to a radial deflection or movement of the lock element, either directly or indirectly via an intermediate member.

The lock element is adapted to block or hold the needle cover in the needle covering position. The lock element may be a deflectable, rotatable or shiftable element, for example in form of a ledge, arm, protrusion or knob. Further, the lock element may be a movable element of the needle cover or of a housing part adapted to contact, abut or engage the needle cover.

The lock element can be in the locking position and may be moved into an unlocking position, intermediate position or end position. If the lock element is in the locking position it preferably abuts a stop surface of the needle cover or of a support or housing element to prevent the needle cover from being moved out of the covering position. If the lock element is not in the locking position it does not prevent the needle cover to be moved out of the covering position. The lock element may be part of the needle cover, of a support element for the needle cover of the housing or any other part of the drug delivery device.

The needle cover in turn is movable along the longitudinal axis and preferably guided within the housing or a member connected to the housing or a member of a reservoir unit attachable to the housing. The movement of the needle cover allows to switch between a safety state in which the needle is covered and the needle does not protrude from the housing and an injection state in which the needle is uncovered and exposes. The needle cover may be implemented as shield or sleeve or sleeve-shaped element adapted to cover or envelop the needle of the drug delivery device the covering position.

The drug delivery device may be an injection device, preferably a disposable, reusable or semi-reusable injection pen, an autoinjector or semi-reusable (or semi-disposable) autoinjector or a patch injector applicable onto the skin of the user for the duration of the injection. Reusable injection devices may include at least a part or a unit that can be reused for several injections. Semi-reusable devices include a unit or part that includes a disposable reservoir with the liquid drug which is discarded after an injection or if the reservoir is empty. The semi-reusable devices further include a reusable part typically including a dispensing mechanism.

Automatic devices or autoinjectors typically include automatic drive means such as an elastic element (for example a preloaded spring) or an electric motor to drive a dispensing member to dispense the liquid drug form the reservoir.

The term "injection device" or "injector" refers to a device that is removed from the injection site after each medication event or drug delivery process. This is in contrast to the term "infusion device" which refers to a device with a cannula or needle that remains in the skin of the patient for a prolonged period of time, for example, several hours.

The term "distal" is meant to refer to the direction or the end of the drug delivery device carrying an injection needle or an injection cannula, whereas the term "proximal" is meant to refer to the opposite direction or end pointing away from the needle or cannula..

In the following description the radial direction is meant to refer to a direction perpendicular to the longitudinal direction or longitudinal axis. The latter is defined by the housing and refers to the direction in which the housing (and preferably the delivery device) has the longest dimension.

In the claims, the word "comprising" does not exclude other elements or steps, and the indefinite article "a" or "an" does not exclude a plurality. For example "an arm" does not exclude the fact that there may be two arms that functionally or structurally fulfill the purpose of "an arm". The mere fact that certain elements or steps are recited in distinct claims shall not preclude the existence of further meaningful combinations of these elements or steps.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances

The safety mechanism includes preferably a counter element and wherein a first guiding surface of the trigger member or the counter element causes the trigger member or the counter element to move the lock element out of the locking position if the trigger member is moved along the longitudinal direction. The counter element may be part of or it may form the above mentioned intermediate member or alternatively the counter element may be a part of the housing of the delivery device.

If the first guiding surface contacts a contact element as a protrusion, cam or counter surface and if the trigger member is further moved along the longitudinal axis the first guiding surface is pressed onto the contact element the guiding surface slips along the counter element such that the longitudinal movement of the trigger member forces the counter element in a radial direction or in any direction inclined to the longitudinal direction

Preferably, the trigger member includes the first guiding surface and the counter element comprises a corresponding second guiding surface adapted to contact and engage the first guiding surface. An interaction of the first guiding surface with the second guiding surface causes the trigger member or the counter element to move the lock element out of the locking position.

In a further preferred embodiment the guiding surface is a first sloped contact surface, preferably arranged on a protrusion, ledge or cam. The second guiding surface is preferably a second sloped contact surface.

The first and the second contact surface are preferably sloped in the same direction or have the same slope angle. The first and second sloped contact surface allow for a reliable actuating of the lock element. Due to the sloped surfaces the longitudinal movement of the trigger member can be reliably transferred to a radial movement or to a movement in an angled direction with respect to the longitudinal axis. In concrete terms, if the first sloped contact surface contacts the second sloped contact surface and if the trigger member is further moved along the longitudinal axis the first sloped surface is pressed onto the second sloped surface thereby causing the two surfaces to slip on each other such that the longitudinal movement of the trigger member forces the counter element in a radial direction or in any direction angled to the longitudinal direction.

The first and the second sloped contact surfaces may be positioned, for example, relative to the longitudinal axis in an angle of at least 20° or in an angle between 40° and 50° to the longitudinal axis.

The lock element is preferably a deflectable arm having an end connected to the needle cover, to the housing or to a holder and the arm has a free end part or end portion adapted to engage a stop surface or counter surface of the needle cover. Thus, if the needle cover is in the covering position the free end part or end portion of the arm engages or abuts the stop surface of the needle cover and thereby prevents a movement of the needle cover out of the covering position towards a retracted position. The lock element in form of a flexible arm allows an easy lock and unlock of the needle cover as the arm can be moved quickly out of the locking position.

Alternatively, the lock element may be a knob or ledge or the like.

As mentioned above the trigger member and the lock element are operatively coupled to each other. Preferably, a movement of the trigger member in a first direction along the longitudinal axis causes the lock element to move radially out of the locking position and a movement of the trigger sleeve in a second, counter direction along the longitudinal axis enables the lock element to move radially into the locking position. Thus, the lock element can be moved radially inwards or outwards, respectively depending on the longitudinal movement direction (distally or proximally) of the trigger member.

The term "move radially" includes movement that have at least a radial movement component. Hence, in case the lock element is a tongue or an arm these elements may swivel or pivot towards and away from the longitudinal axis and such a movement shall be encompassed by the term "move radially".

In case the safety mechanism is used in a drug delivery device with an electric motor the motor can be driven forward and backwards to move the trigger member in either direction. Therefore, a simple and reliable locking or unlocking of the needle cover can be provided.

The drug delivery device preferably comprises a reservoir holder for holding the reservoir with the liquid drug. The needle cover is preferably movable relative to both the lock element and the reservoir holder if the needle cover is moved out of the covering position. In this embodiment the needle cover may be engaged and guided by the reservoir holder.

The safety mechanism preferably includes a sleeve-shaped support member for holding the reservoir or a reservoir holder encompassing the reservoir. The support member may be arranged proximally or/and outside the trigger member and the support member has preferably a non-circular cross section. That provides a roll-away protection for the drug delivery device.

In a preferred first embodiment the safety mechanism includes an intermediate member (or connecting element) which is preferably a sleeve and which comprises the counter element with a second guiding surface (or a second sloped contact surface). In this embodiment a movement of the trigger member along the longitudinal axis causes the first guiding surface (or first sloped contact surface) of the trigger member to interact with the second guiding surface (second sloped contact surface) of the intermediate member. This moves a portion of the intermediate member in a first radial direction or in an inclined direction thereby causing the lock element to move out of the locking position. Preferably, the intermediate member contacts or abuts the lock element and the lock element is moved by the intermediate member in a radial direction or inclined direction. Thus, the longitudinal movement of the trigger member is redirected to a radial movement of a portion of the intermediate member. This allows for a space-saving design as the trigger member is moved longitudinally and the intermediate member is moved radially. The dimensions of the safety mechanism in a radial direction are thus reduced compared to a mechanism with a radially moving trigger member.

In this embodiment the trigger member does not directly contact the lock element but the intermediate member is operatively positioned between the trigger member and the lock element. That means the lock element is controlled via intermediate member which allows to transfer a movement of the trigger member by means of a gear ratio or the intermediate member can be used to selectively transfer or not transfer movements of the trigger member to the lock element. Therefore, a sophisticated kinematic control structure can be provided.

Preferably, the trigger member with the guiding surface or first sloped contact surface is exclusively moved along the longitudinal axis whereas the intermediate member with the second guiding surface or second sloped contact surface is exclusively moved in a radial direction or in a direction angled to the longitudinal axis. By preference, the intermediate member abuts, contacts or engages the lock element to move it in or out of the locking position.

The intermediate member is preferably an arm, cam or protrusion. The intermediate member may be at least partially arranged coaxially inside the trigger member, preferably it is radially arranged between the lock element and the trigger member. This allows for a space-saving design.

Preferably, the arm cam or protrusion is connected to a sleeve-shaped member forming the intermediate member. In this embodiment the intermediate member has an asymmetric cross section in a plane perpendicular to the longitudinal axis. That means the intermediate member may have a movable portion or a movable element (in form of the mentioned arm, cam or protrusion) on one side or on a several sides. The movable portion may be adapted to engage the trigger member and/or the lock element. Preferably, the second guiding surface (second contact surface) is arranged on or integrally formed in the movable portion of the intermediate member.

The trigger member includes by preference further a third guiding surface and the intermediate member comprises a fourth guiding surface. A movement of the trigger member in a second, counter direction along the longitudinal axis causes the third guiding surface to engage the fourth guiding surface and thereby moving a portion of the intermediate member in a second radial direction allowing the lock element to move from an unlocking or intermediate position into the locking position.

Therefore, by means of the third and fourth guiding surfaces the lock element can be easily and reliably moved into the locking position (thereby locking the needle cover) and by means of the first and second guiding surface the lock element can be moved out of the locking position (thereby releasing the needle cover).

As described above with respect to the first and second surface also the third and fourth surface may interact with each other such that the longitudinal movement of the trigger member is transferred to a lock element radial movement or a lock element movement in a direction angled to the longitudinal axis.

The third guiding surface is preferably implemented as third sloped contact surface and the fourth guiding surface is preferably a fourth sloped contact surface.

Instead of or additionally to the above the intermediate member (or a portion of the intermediate member) may be resilient or elastic such that the intermediate member can be deflected or biased and returns to an initial state due to the elastic properties.

The intermediate member (or a portion thereof) may be biased by the trigger member movement in the first longitudinal direction when the first guiding surface interacts with the second guiding surface and the intermediate member may return back or deflect back to the initial state by the trigger member movement in the second longitudinal direction when the first and second guiding surfaces disengage from each other.

The biasing feature enables the intermediate member to switch back or move back to the initial state without additional interaction. This allows to ensure that intermediate member does not unintentionally move the lock element out of the locking position if, for example, the intermediate member is biased in a state in which the lock element is in the locking position.

By preference, the intermediate member includes a radially protruding actuation element in form of a cam, nose, lobe or protrusion. The control element is adapted to interact with and actuate the lock element. Depending on the form and the design of the actuation element a specific movement of the trigger member can be transferred to the lock element. Hence, the design of the actuation element defines how the lock element is actuated and how the lock element is moved by the intermediate member. The actuation element includes a contact portion which is adapted to contact the lock element to move the lock element into or out of the locking position.

Furthermore, the trigger member includes a control element wherein the first and third guiding surface (third contact surface) are arranged each on an opposite side of the control element in a longitudinal direction. The control element may be implemented as cam or protrusion.

The control element may protrude, for example, in a radial direction, e.g. in a plane perpendicular to the longitudinal axis. The control element may contact and engage the second and/or fourth guiding surface of the intermediate member if the trigger member is moved along the longitudinal direction. The control element allows a simple controlling and transfer of movement from trigger member to the intermediate member.

The second and fourth guiding surface are preferably arranged on an intermediate control element (cam or protrusion), which is preferably directed in a radial direction too. Therefore, the interaction between the trigger member and the intermediate member is implemented via control element and intermediate control element again allowing for a space-saving design.

The safety mechanism according to invention comprises in a preferred embodiment a cap releasably attached at a distal end of the needle cover in an initial state or unused state of the drug delivery device. In this embodiment the needle cover or the cap includes a resilient and deflectable element (tongue, arm, protrusion or the like) holding the cap on the needle cover in a non-deflected state. A cap actuation element is operatively (direct or indirect) coupled to both the trigger member and the deflectable element. A movement of the trigger member in the longitudinal direction causes a movement of the cap actuation element which in turn causes a change of the deflectable element from the non-deflected state to a deflected state thereby releasing the cap from the needle cover. That means after the deflectable element is deflected the user can remove the cap from the needle cover to prepare the delivery device for an injection.

The deflectable element may be separate from the needle cover or from the cap or it may be integrally formed (monolithic) in the needle cover or in the cap. The deflectable feature allows to connect the cap easily by a releasable snap-fit connection.

In a preferred embodiment the intermediate member includes the cap actuation element (e.g. the cap actuation element may be integrally formed in the intermediate member) and the trigger member comprises further a first cap surface and the intermediate member comprises a second cap surface which are both sloped surfaces, wherein the movement of the trigger member in the first direction along the longitudinal axis causes the first cap surface to engage the second cap surface thereby moving the intermediate member (or a portion thereof) in a first radial direction which causes the deflectable element to deflect.

The trigger member includes by preference further a third cap surface and the intermediate member comprises a fourth cap surface which are both sloped surfaces. A movement of the trigger member in a counter second direction along the longitudinal axis causes preferably the third cap surface to interact with the fourth cap surface thereby moving the intermediate member (or a portion thereof) in a second radial direction allowing the deflectable element to deflect back in its initial position.

The intermediate member includes in this embodiment not only an actuation element for moving the lock element but also a cap actuation element, preferably in form of a protrusion, nose, lobe or cam for moving the deflectable element. That means the intermediate member provides two functions: actuating the lock element (for holding or releasing the needle cover) and actuating the deflectable element (for holding or releasing the cap). Hence, two function can be provided with one single member. For that purpose, the intermediate member may comprise two independently deformable portions, a first deformable portion for actuating the lock element and a second deformable portion for actuating the deformable element for the cap.

In a second preferred embodiment of the safety mechanism according to the invention the housing includes the counter element. Thus, in contrast to the above mentioned first embodiment no intermediate member is present and the counter element is integrally formed in the housing or in a member fixedly connected to the housing.

The trigger member may include a body and a deflectable portion comprising the first guiding surface (sloped contact surface) and movement of the trigger member body in the first direction along the longitudinal axis causes the first guiding surface (sloped contact surface) to interact with the housing thereby moving the deflectable portion of the trigger member in a first radial direction or in a direction inclined with respect to the longitudinal axis thereby engaging and moving the lock element out of the locking position towards a non-locking position. Hence, the trigger member directly contacts and engages the counter element without any intermediate member.

Optionally, the housing includes a second guiding surface adapted to interact with the first guiding surface.

As described above with respect to the first embodiment when the lock element is moved out of the locking position the needle cover can be moved out of the covering position and thus exposing the needle. If the housing features the second guiding surface a simple design is provided as no further member or part is required. The first and second guiding surfaces are preferably a first and second sloped contact surface.

The deflectable portion of the trigger member may be, for example, implemented as arm, tongue or deflectable protrusion. The deflectable portion is preferably and essentially moved in a first radial direction or in a direction angled to the longitudinal axis by the longitudinal movement of the body of the trigger member.

In second embodiment the safety mechanism preferably includes a cap releasably attached to a distal end of the needle cover in an initial state, shipping state or unused state of the delivery device. The cap or the needle cover includes a deflectable element holding the cap on the needle cover, preferably in a non-deflected state. A movement of the needle cover in the longitudinal direction out of the covering position towards a retracted position causes a change of the deflectable element from the non-deflected state to a deflected state thereby releasing the cap from the needle cover.

In a preferred embodiment an automatic movement of the needle cover and cap relative to the housing releases the cap from the needle cover.

The cap can thus be hold on the needle cover by a releasable snap-fit connection. Furthermore, a simple longitudinal movement of the needle cover may release the connection and the cap can be removed and the delivery device is ready for an injection.

The invention relates further to an injection device for dispensing the liquid drug from the reservoir. The injection device comprises a plunger rod and an automatic drive for driving the plunger rod to move a piston inside the reservoir to dispense the liquid drug form the reservoir through the injection needle or cannula. The injection device further includes the safety mechanism as described above.

The injection device may be, for example, a manual user-driven or automatic injection pen, a autoinjector including a spring element or motor for automatically driving the plunger rod or a semi-reusable injection device or semi-reusable autoinjector. Such reusable devices include a reusable drive unit and a disposable reservoir unit or syringe unit which is releasably attachable (for example by a thread or bayonet connection) to the drive unit.

The automatic drive is by preference adapted to rotate a drive member of the drive unit, wherein the drive member is operatively coupled (direct or indirect) to both the plunger rod and the trigger member, wherein rotation of the drive member by the automatic drive in a first rotational direction causes the plunger rod and the trigger member to be moved in the first direction along the longitudinal axis and rotation of the drive member in a second, counter rotational direction causes the plunger rod and the trigger member to be moved in the second direction along the longitudinal axis.

Hence, with one single automatic drive the plunger rod can be moved back and forth and preferably the trigger member can be moved back and forth at the same time. That allows to lock and unlock the needle cover (via lock element) and, if any, lock and release a cap on the cover sleeve (via intermediate member, if any) and with the same automatic drive the plunger rod can be driven to dispense the drug form the reservoir. That means the automatic drive can be used to rotate the drive member to move the trigger member, preferably before an injection to lock or unlock the cover member and potentially the cap and subsequently the automatic drive can be used to dispense the drug during an injection or for preparation steps.

Preferably, the drive member is in treaded engagement with the plunger rod, such that the plunger rod is exclusively shifted along the longitudinal direction when the drive member is rotated. The trigger member is preferably directly or indirectly coupled to the plunger rod such that a longitudinal movement of the plunger rod in the first direction causes to move the trigger member in the longitudinal direction in the first direction and movement of the plunger rod in the second longitudinal direction causes to move the trigger member in the second longitudinal direction.

The injection device further comprises preferably a reservoir unit (or syringe unit) and a drive unit. The reservoir unit is preferably releasably attachable to the drive unit. The reservoir unit comprises a reservoir for the liquid drug (e.g. a cartridge, a syringe or a container), the needle cover and the lock element and the drive unit includes the housing, the automatic drive, the plunger rod, the trigger member and, if any, the intermediate member.

The reservoir unit is preferably disposable and the drive unit is preferably reusable. That means the injection device comprises two units allowing to establish a semi-reusable concept which reduces the waste. In contrast to conventional injection devices the semi-reusable device does not have be discarded completely but only the reservoir unit can be disposed after use or if the drug is fully dispensed out of the reservoir. The drive unit can be reused for several injections.

The reservoir unit is releasably attachable to the drive unit. That means the two units can be detached and reattached without destroying elements. Non-limiting examples of releasable connections are a snap-fit, a bayonet connection or a thread.

In a preferred embodiment the reservoir unit includes a radially extending protrusion or radially extending rim portion and the drive unit includes a radially deflectable and flexible holding arm or clamping arm adapted to engage the protrusion or rim portion such that the syringe unit is hold by the drive unit via snap-fit connection.

The drive unit features preferably and additionally a lock to lock the snap-fit connection such that the syringe unit cannot be removed from the drive unit if the lock is activated. The lock may be implemented by the above described lock element or/and by a separate lock element preventing, for example, a radial movement of the holding arms and thereby locking the arms relative to the protrusion or rim portion.

Preferably, the reservoir inside the reservoir unit is a prefilled syringe with a non-releasable connected injection needle at the distal end thereof.

By preference, upon attachment the reservoir unit is held by the drive unit relative to the housing such that a movement of the reservoir unit in a dispensing direction is prevented.

The invention further refers to a method for a safety mechanism for an injection device for dispensing a liquid drug from a reservoir through a needle. The method includes the steps of
a. Providing an injection device with a housing defining a longitudinal axis;
b. Moving a trigger member inside the housing along the longitudinal direction
c. Moving a lock element, by the trigger member as the trigger member is operatively coupled to the lock element, out of a locking position in which the lock element engages a needle cover to prevent the needle cover from being moved out of a needle covering position,
d. Releasing, by the movement of the lock element, the needle cover from the covering position and moving the needle cover out of the needle cover position towards a retracted position in which the needle is exposed.

### BRIEF DESCRIPTION OF THE DRAWINGS

The subject matter of the invention will be explained in more detail in the following text with reference to preferred exemplary embodiments which are illustrated in the attached drawings, in which:
- Fig.1: depicts a perspective view of a semi-reusable autoinjector according to the invention;
- Fig.2: depicts an exploded view of the syringe unit of the autoinjector from figure 1;
- Fig.3: depicts a sectional view of the drive unit of the autoinjector;
- Fig. 4: depicts the drive unit with an inserted syringe unit;
- Fig. 5: depicts a sectional view of the autoinjector where the syringe unit is locked to the drive unit;
- Fig. 6a: depicts a syringe unit according to a second embodiment;
- Fig. 6b: depicts the syringe unit of fig. 6a but without cap;
- Fig. 7: depicts a sectional view of a drive unit of the second embodiment;
- Fig. 8: depicts a perspective view of a gripper sleeve of the drive unit of the second embodiment;
- Fig. 9a: depicts the drive unit with an inserted syringe unit according to the second embodiment;
- Fig 9b: depicts a sectional view of control cams for unlocking a cover sleeve and cap cams for unlocking a cap;
- Fig. 10: depicts the cap cams in a locking state;
- Fig. 11: depicts a the control cams as well as the cap cams in a locking state;
- Fig. 12: depicts the control cams and the cap cams during an injection;
- Fig. 13: depicts the autoinjector after release of the syringe unit and
- Fig. 14: depicts a third embodiment of the autoinjector;

The reference symbols used in the drawings, and their primary meanings, are listed in summary form in the list of designations. In principle, identical parts are provided with the same reference symbols in the figures.

### DETAILED DESCRIPTION OF PREFERRED EMBODIMENTS

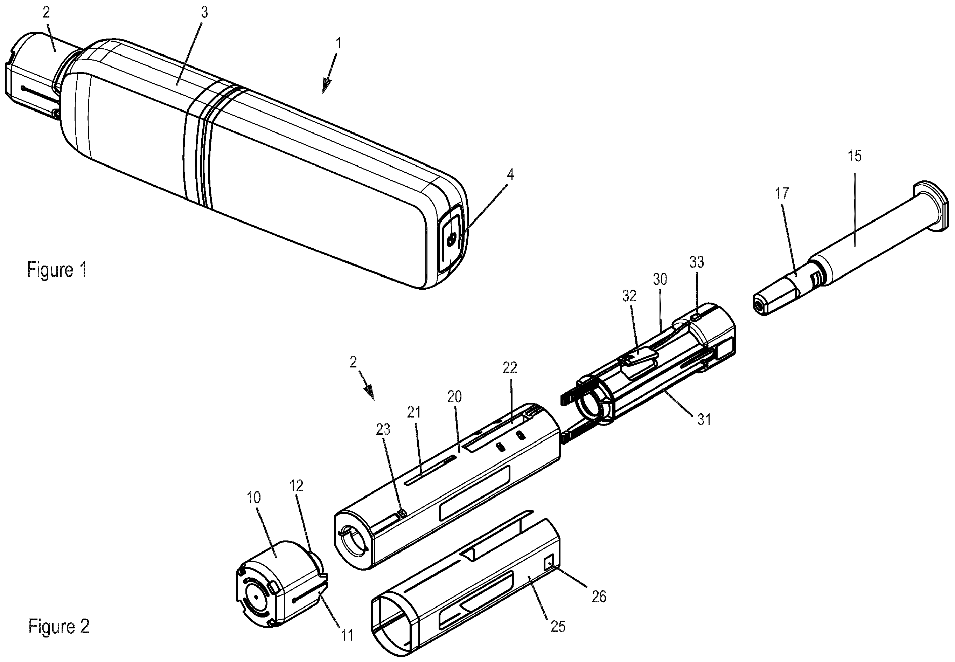

In the present description the term "distal" refers to the side where the injection needle is located. This is on the left hand side in the figures 1 to 13. The term "proximal" refers to the opposite side or rear end of the device and is on the right hand side in figures 1 to 13.

First embodimentFigure 1 shows a perspective view of a drug delivery device of a first embodiment of the invention in form of a semi-reusable autoinjector 1. The autoinjector 1 comprises a disposable syringe unit 2 and a reusable drive unit 3. As shown in figure 1 the drive unit 3 has an elongated housing defining a longitudinal axis and comprises a button 4 at its proximal end.

In the following the structural features of the syringe unit and the drive unit will be descripted in detail. Subsequently, the function of the semi-reusable autoinjector 1 will be explained.

Figure 2 depicts an exploded view of the syringe unit 2. As shown in figure 2 the syringe unit 2 includes a syringe holder 30, a cover sleeve 20 coaxially arranged around the syringe holder 30 and movable relative to the syringe holder along the longitudinal axis, a label 25 wrapped around the cover sleeve 20, a cap 10 and a prefilled syringe 15 comprising a liquid drug and a rigid needle shield 17 on the injection needle. The syringe holder 30, the cover sleeve 20 and the cap 10 are individually injection-molded plastic parts.

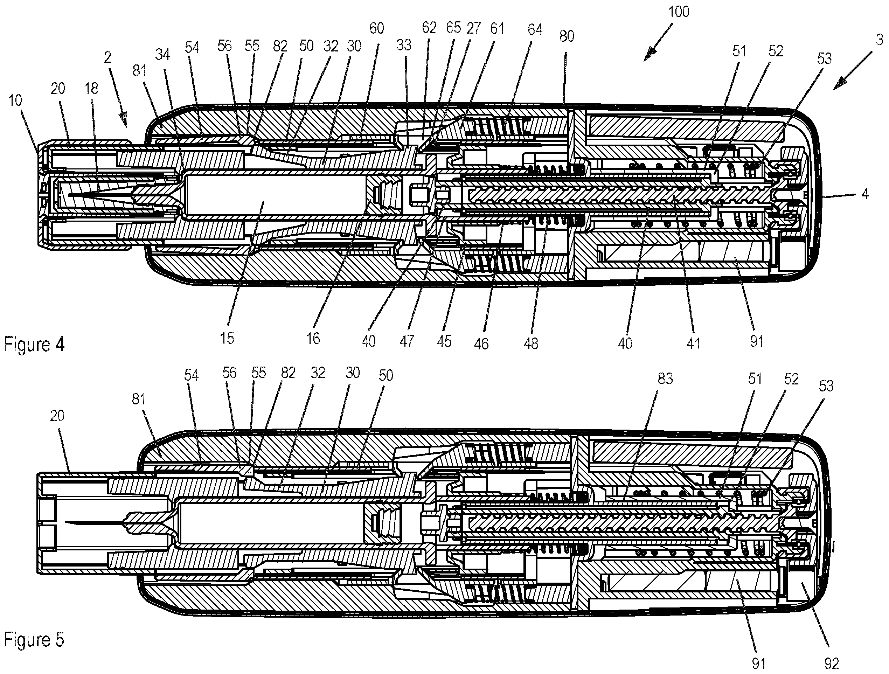

The prefilled syringe 15 with a barrel made of glass is rotationally and axially fixed inside the sleeve-shaped syringe holder 30. For that purpose, the syringe holder 30 includes clamping elements (not shown) pressing on an outer surface of the syringe barrel and a distal shoulder of the syringe abuts a bearing surface 34 (see figure 4) inside the syringe holder 30. As shown in figure 2 the syringe holder 30 further features longitudinal guiding rails 31 on its outside for the cover sleeve 20 such that the cover sleeve 20 can be shifted exclusively in the longitudinal direction relative to the syringe holder 30. Furthermore, the syringe holder 30 comprises two radially deflectable lock elements in form of locking arms 32 arranged on opposite sides of the syringe holder 30. In an initial state the locking arms 32 are in a non-deflected state and in a locking position and prevent a movement of the cover sleeve 20 relative to the syringe holder 30 in a proximal direction. Hence, the cover sleeve 20 shields or covers the injection needle of the syringe 15 in a covering position. For that purpose, a free end of the locking arms 32 abut stop surfaces (not shown) inside the cover sleeve 20. As will be descripted below once the locking arms 32 are deflected radially inwards the cover sleeve 20 can be pushed proximally. The syringe holder 30 further includes two oppositely arranged and radially protruding ledges 33 at a proximal end of the syringe holder 30 for engagement with clamping arms 61 (figure 4) inside the drive unit 3.

The cover sleeve 20 has a non-circular cross section in a plane perpendicular to the longitudinal axis. That means two sides of the cover sleeve are flattened. The cover sleeve comprises a first lateral opening 21 for access to the locking arms 32 and a proximal second opening 22 for access to the ledges 33 from outside. The label 25 wrapped around the cover sleeve 20 includes a machine-readable tag (not shown) in form of a RFID or QR code 26 identifying the syringe unit 2.

At a distal end of the cover sleeve 20 the cap 10 is releasably attached by a snap-fit connection. The cap 10 includes on two sides two deflectable elements or tongues 11 and on each side a protrusion engaging in a corresponding indentation 23 in the cover sleeve 20 for the snap-fit connection. Alternatively, the cap 10 may have an ellipse shape in a plane perpendicular to the longitudinal axis wherein inside the ellipse form the protrusions engage the indentation in the cover sleeve. The ellipse can be deformed to a circular shape to release the protrusions from the indentation 23. The release of the snap-fit will be descripted below with respect to the decapping of the syringe unit 2. The cap 10 includes in its inside a sleeve-shaped holder 12 connectable to the rigid needle shield (RNS) 17 of the syringe 15 such that the RNS 17 is removed with the cap 10 if the cap is released from the needle cover.

The syringe 15 inside the syringe holder 30 has a barrel made of glass and inside the barrel a movable piston 16 (figure 4). An injection needle 18 (figure 4) is non-releasably connected to a distal end of the syringe barrel. In an unused state the injection needle 18 is covered by the RNS 17. The latter in turn is axially and radially fixedly connected to the cap 10 by the holder 12 once the syringe 15 is mounted to the syringe holder 30.

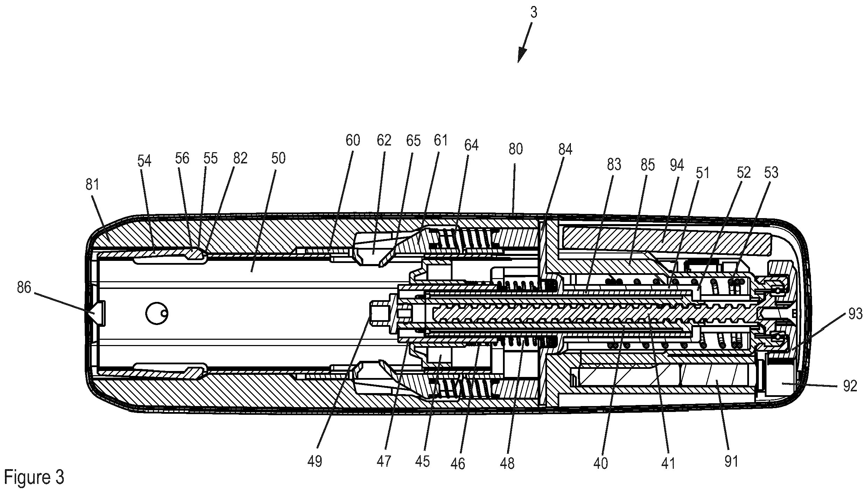

Figure 3 depicts a sectional view of a first embodiment of the drive unit 3. The cut of the sectional view runs along the longitudinal axis of the device. The drive unit 3 comprises a housing 80 including an outer housing cover and inside the cover a mechanics sleeve 83 comprising a radial wall 84 that separates the drive 3 unit in a distal portion and a proximal portion. The mechanics sleeve 83 is immovably connected to the housing 80. Inside the housing 80 and distally the separating wall 84 is a distal housing insert 81 whereas proximal the wall 84 a proximal housing insert 85 is located. The distal and proximal housing inserts 81, 85 are immovably connected to the housing 80. Furthermore, the drive unit 3 includes an opening on its distal side adapted to accommodate a proximal portion of the syringe unit 2.

Inside the distal housing portion and guided by the distal housing insert 81 a gripper sleeve 60 is arranged and movable relative to the housing 80 along the longitudinal axis. Coaxially to the gripper sleeve 60 a trigger sleeve 50 is arranged. Again inside the trigger sleeve 50 a cover sleeve connector 45 is located and biased in distal direction by a cover sleeve spring 46. The spring abuts on its distal end the cover sleeve connector 45 and on its proximal end the radial wall 84 of the mechanics sleeve 83. A sleeve-shaped syringe connector 47 is coaxially arranged inside the cover sleeve connector 45 and biased distally by a syringe connector spring 48 coaxially inside the cover sleeve spring 46 and proximally supported by the wall 84. The spring 46 ensures that the syringe connector 45 pushes the syringe 15 of an inserted syringe unit 2 in distal direction.

The proximal housing portion accommodates the proximal housing insert 85 which supports and guides a sleeve-shaped trigger sleeve connector 51 movable along the longitudinal axis, a drive assembly with an electric motor 91, a pinion 92 and a gear 93 connecting the motor 91 with a threaded rod 41, a battery 94, the treaded rod 41 and a plunger rod 40 in threadedly connected to the threaded rod 41. The proximal housing portion further accommodates an electronic module (not shown) with a controller configured to control the electronic motor 91 and providing information to the user via a display or LEDs (not shown) or a communication module (not shown). The trigger sleeve connector 51 is fixedly and immovably connected to the trigger sleeve 50 and distally pressed onto a proximal end of the plunger rod 40 by a trigger sleeve spring 53. The plunger rod 40 non-rotatably guided by the mechanics sleeve 83 and comprises on its distal end a flange 49 adapted to engage the piston 16 (see figure 4) during dispensing.

Inside the proximal housing portion is further an axially arranged detection pin (not shown) axially movable by the cover sleeve connector 45. The detection pin is connected to and interacts with a TMR position sensor. The presence of a syringe unit 2 inside the drive unit housing 80 is thus detectable via detection pin and the position sensor which provides a corresponding signal to the controller of the electronic module.

The gripper sleeve 60 includes two axially extending and deflectable clamping arms 61 having a clamp 62 at their free distal end adapted to accommodate the syringe holder ledges 33. The clamping arms 61 can be deflected radially outwards allowing the clamps 62 to snap onto the ledges 33 of the syringe holder 30 when the syringe unit is inserted into the opening in the drive unit 3. On its outside the gripper sleeve 60 comprises splines (not shown) running in corresponding longitudinal grooves (not shown) in the distal housing insert 81 guiding the gripper sleeve 60 during an axial movement relative to the housing 80. Furthermore, each clamping arm 61 provides a support for an axially aligned gripper sleeve spring 64 which biases the clamping arms 61 in a holding position.

The trigger sleeve 50 is axially guided by the gripper sleeve 60 as well as by the distal housing insert 81 and shiftable relative thereto. For this purpose, the trigger sleeve 50 includes longitudinal guiding rails (not shown) engaging longitudinal grooves (not shown) in both the distal housing insert 81 and the gripper sleeve 60. As it can be seen in figure 3 the trigger sleeve 50 incudes oppositely arranged trigger arms 54 which include on their proximal free end 56 a guiding surface or sloped contact surface 55. The trigger arms 54 are deflectable radially inwards upon contact of the contact surface 55 with a counter guiding surface or counter sloped contact surfaces 82 of the distal housing insert 81. As mentioned above the trigger sleeve 50 is further immovably connected to the trigger sleeve connector 51 in the proximal housing portion. As the trigger sleeve connector 51 is biased by the trigger sleeve spring 53 the trigger sleeve 50 is biased too in distal direction. The trigger sleeve connector 51 in turn is connected to the plunger rod via ledges 52 which engage a proximal shoulder of the plunger rod 40 such that an axial movement of the plunger rod 40 in proximal direction moves also the trigger sleeve 50 proximally.

In the state shown in figure 3 the drive unit 3 is ready to be loaded with a syringe unit 2. The controller displays the current state to the user via display by showing a message like "Syringe Unit Insertion" or via green or red LEDs.

In order to prepare the autoinjector 1 for an injection the user attaches a syringe unit 2 to the drive unit 3. The electronics in the drive unit 3 are switched from an inactive state or sleep mode to an active mode. Alternatively, the user can press the button 4 to activate the electronics.

Figure 4 depicts a sectional view of the drive unit 3 with an inserted syringe unit 2. The syringe unit 2 has been inserted with its proximal end portion into the distal opening of the drive unit housing 80. In the state shown in figure 4 the proximal ledges 33 of the syringe holder 30 are snapped into recesses of the clamps 62 of the gripper sleeve clamping arms 61. The syringe unit 2 is thus held in the drive unit 3 by the clamping arms 61. However, in the shown state in figure 4 the syringe unit 2 can still be pulled out of the drive unit housing by overcoming a holding force. Hence, if the user pulls the syringe unit 2 in a distal direction the syringe holder 30 with the syringe 15 are firstly moved in distal direction relative to the cover sleeve 20 until stop and subsequently the cover sleeve 20 is moved distally too. This brings inclined release surfaces 27 of the cover sleeve 20 into contact with inclined counter surfaces 65 of the clamping arms 61. If the cover sleeve 20 is further pulled in distal direction the clamping arms 61 are deflected radially outwards via release surfaces 27 and counter surfaces 65. The ledges 33 of the syringe holder 30 are therefore released from the clamping arms 61 and the syringe unit 2 can be removed from the drive unit 3. out of the drive unit 3 the clamping arms 61 may deflect radially outwards and release the ledges 33.

That means in the state shown in figure 4 the syringe unit 2 is held inside the drive unit 3 but not yet locked inside the drive unit 3.

Figure 5 depicts the autoinjector 1 in a locked state without cap. When the syringe unit 2 is inserted as described above the syringe unit 2 moves the detection pin and thus the position sensor detects the movement and automatically sends a signal to the controller in the electronic module which detects the presence of the syringe unit 2 inside the drive unit housing. A code reader (not shown) in form of an NFC reader or QR code reader inside the drive unit 3 reads the RFID or QR code 26 of the syringe unit 2. Subsequently, the controller of the drive unit sends the read medication information from the read code 26 to an external device to verify the drug or medication with predefined information. Alternatively, the controller compares the read medication information from the code 26 with predefined information stored in a memory inside the drive unit 2.

In case of successful medication verification the controller receives an enable signal (either from the external device or from the internal comparison). The controller then controls the electric motor 91 to rotate the threaded rod 41 to move the trigger sleeve 50 a short distance of several millimeters in proximal direction. Such a trigger sleeve 50 movement is achieved by a kinematic chain as described as follows.

The motor 91 rotates the pinion 92 and the gear 93 transfers the rotation to the threaded rod 41 which in turn moves the non-rotating plunger rod 40 in proximal direction. As the trigger sleeve connector 51 is connected via its ledges 52 to a proximal shoulder of the plunger rod 40 the latter causes the trigger sleeve connector 51 to move and thus the trigger sleeve 50 is shifted in proximal direction too.

The proximal movement of the trigger sleeve 50 brings the sloped contact surfaces 55 of the trigger arms 54 into contact with the counter sloped contact surfaces 82 of the distal housing insert 81. As the two sloped surfaces 55, 82 slide along each other the trigger arms 54 are forced to deflect radially inwards when the trigger sleeve 50 is further moved in proximal direction. The free ends 56 of the deflected trigger arms 54 in turn come into contact with the locking arms 32 of the syringe holder 30. Subsequently, the trigger arms 54 deflect the locking arms 32 radially inwards and thus move the locking arms 32 out of a locking position. As a consequence, the locking arms 32 no longer engage the stop surface of the cover sleeve 20 and thus allow for the cover sleeve 20 to move out of a covering position and move inside the housing 80 in proximal direction relative to the syringe holder 30 and relative to the drive unit housing 80.

At the same time the radially inwards deflected trigger sleeve arms 54 abut a distal stop surface (not shown) of the syringe holder 30 and thereby lock the syringe holder 30 relative to the trigger sleeve 50 such that a movement of the trigger sleeve 50 is transferred to the syringe unit 2. Thus, in this state (figure 5) the syringe unit 2 can no longer pulled out of the drive unit 3.

The controller displays the locked state to the user via display. As in this state the cap is still mounted onto the distal end of the cover sleeve 20 the next step is the decapping which may be started by pressing the button 4. The controller then drives the motor 91 to move additionally the trigger sleeve 50 a short distance of 1 to 5 mm in proximal direction and immediately the same distance back in distal direction. As the trigger arms 54 fix the syringe unit 2 relative trigger sleeve 50 the syringe unit 2 (and the gripper sleeve 60) are moved back and forth too. The proximal movement of the syringe unit 2 with the cap 10 relative to the drive unit housing 80 compresses the trigger sleeve spring 53. In figure 5 it can be seen the trigger sleeve connector 51 was moved a distance in proximal direction (see distance between ledges 52 and proximal end of mechanics sleeve 83). The proximal movement of the syringe unit 2 relative to the housing 80 further causes the deflectable cap tongues 11 (figure 2) of the cap 10 to abut against a triangle-shaped protrusion 86 (figure 3) on a distal end of the drive unit housing 80. As a consequence, if the syringe unit 2 is further moved into the drive unit housing 80 the protrusion 86 deflects the cap tongues 11 and thereby releases the snap-fit connection of the cap 10 with the cover sleeve 20. Consequently, the cap 10 is wiped off. This leads to the state shown in figure 5. The controller moves then motor 91 in counter direction to move the plunger rod 40 back the short distance of 1 to 5 mm but without unlocking the syringe unit 2. The syringe unit 2, the trigger sleeve 50 and the gripper sleeve 60 are moved back due to the spring force of the trigger sleeve spring 53.

The autoinjector 1 is now decapped and ready for an injection. The controller displays a corresponding notification on the display.

As a next step the user places the distal end of the cover sleeve 20 onto an injection site and pushes the autoinjector 1 towards the injection site. This causes the cover sleeve 20 to move proximally from the distal covering position into the drive unit housing 80 (push on skin) and compresses the cover sleeve spring 46 which biases the cover sleeve 20 in distal direction via cover sleeve connector 45. Upon push on skin the cover sleeve connector 45 is moved proximally and thus the detection pin is further moved proximally. This further movement is detected by a further TMR position sensor which triggers the controller in the electronic module to start the injection. That means the controller drives the electric motor 91 in dispensing direction and thus rotates the threaded rod 41 and thereby moves the plunger rod 40 in distal direction. As the trigger sleeve spring 53 biases the trigger sleeve connector 45 on the proximal shoulder of the plunger rod 40 (alternatively the trigger sleeve connector 45 is immovably connected to the plunger rod 40) the trigger sleeve 50 is also moved in distal dispensing direction. The distal flange 49 of the plunger rod 40 moves the piston 16 inside the syringe 15 in distal direction and thus dispenses liquid drug through the injection needle out of the syringe 15.

The distal movement of the plunger rod 40 moves the trigger sleeve via trigger sleeve connector 51 in distal direction to its initial position. That means the trigger arms 54 can move radially outwards back into their non-deflected position. However, as the cover sleeve 20 is in a retracted proximal position the locking arms 32 are prevented from being moved back into the locking position.

The controller displays a holding time indicating a time period during that the user has to hold the autoinjector 1 onto the injection side after the injection to ensure that the drug is completely administered and absorbed. The controller may display a down counter informing the user about the remaining holding time. Once the holding time is elapsed a notification is shown to the user that the autoinjector can be removed from the injection site. Alternatively, the end of the holding time may be indicated only by a LED.

When the user removes the autoinjector 1 from the injection site compressed cover sleeve spring 46 can release and thus it can move the cover sleeve 20 back distally into the needle covering position.

During dispensing the trigger sleeve 50 was moved in its initial distal and unlocking position in which the trigger arms 54 are not deflected anymore and thus do not contact the locking arms 32 of the syringe holder 30. That means the locking arms 32 are no longer completely pressed radially inwards and upon movement of the cover sleeve 20 into its covering position the locking arms 32 are able to switch back into their locking position in which the free end of the arms 32 abuts the contact surface inside the cover sleeve 20 thereby locking the cover sleeve 20 in its covering position as in the state shown in figure 4.

Furthermore, as the trigger arms 54 are not deflected anymore and thus do not contact the distal stop surface of the syringe holder 30 the entire syringe unit 2 can be pulled out of the drive unit housing 80 by the user. The user can now discard the syringe unit 2 and the drive unit 3 is ready to be loaded with a new syringe unit.

### Second embodiment

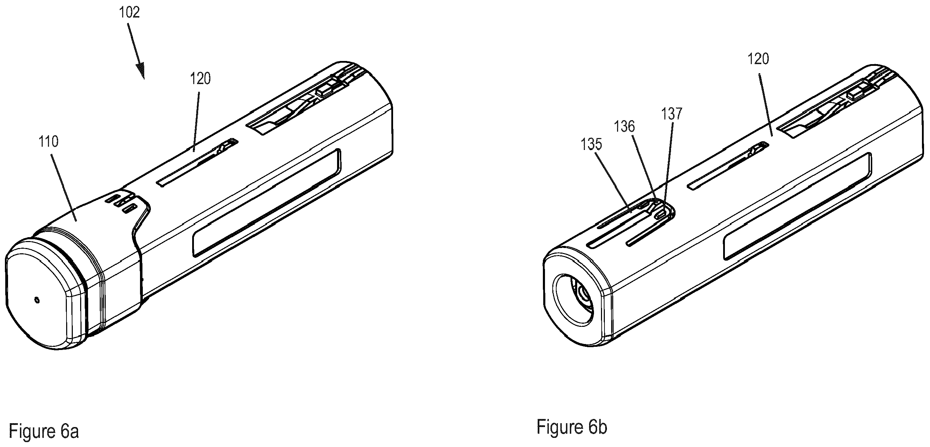

In the following a second embodiment of the present invention is descripted with respect to figures 6a to 13. The drug delivery device of the second embodiment is a semi-reusable autoinjector 100 including a drive unit 103 and a syringe unit 102. The differences to the first embodiment autoinjector will be descripted in detail.

The syringe unit 102 of the second embodiment is shown in figure 6a and 6b is similar to the syringe unit of the first embodiment. Figure 6a depicts a perspective view of the syringe unit 102 with the cap 110 and figure 6b depicts the syringe unit 102 without cap.

In contrast to the first embodiment the syringe holder 130 of the syringe unit 102 of the second embodiment includes two cap holding tongues 135 extending axially and deflectable radially inwards. The two oppositely arranged tongues 135 have each a free end including a radially outwards extending protrusion 136 adapted to interact with a cap actuation nose 179 as described below. Each tongues 135 further has two ledges 137 next to protrusion 136 on both sides. The ledges 137 engage corresponding recesses in the cap 110 to hold the cap 110 on the cover sleeve 120 by a releasable snap-fit connection.

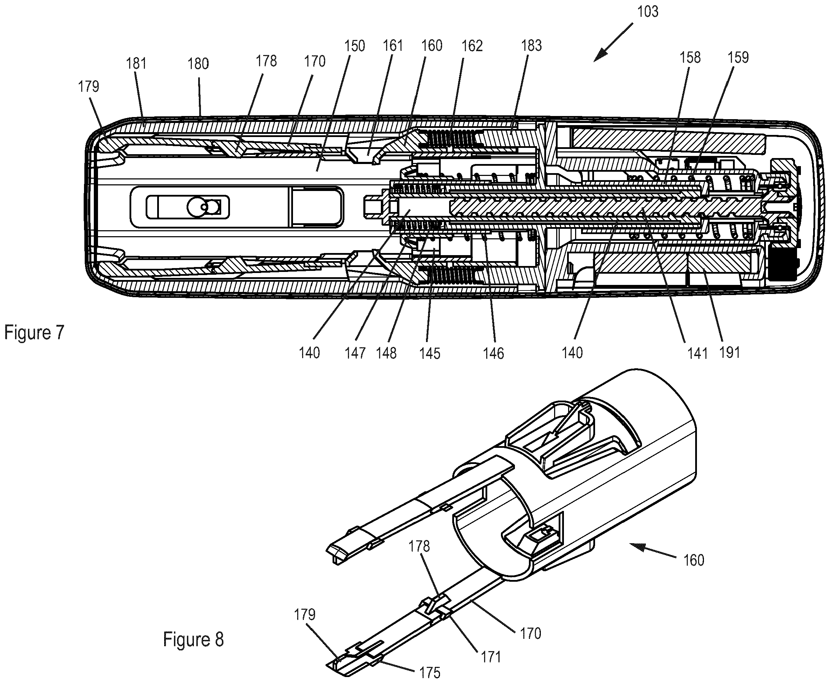

Figure 7 depicts a sectional view of a drive unit 103 of the second embodiment. In contrast to the first embodiment described above the drive unit 103 includes an intermediate sleeve 160 radially positioned between a distal housing insert 181 and a trigger sleeve 150.

Figure 7 depicts a perspective view of the intermediate sleeve 160. As shown in figure 7 the intermediate sleeve 160 comprises two axially extending levers 170 that are connected to a sleeve-shaped element similar to the above descripted gripper sleeve of the first embodiment. The intermediate sleeve 160 further includes two clamping arms 161 (see figure 7) for holding the syringe unit 102 as descripted with respect to the first embodiment. Each clamping arm 161 is connected to a spring 162. The spring 162 is supported on a mechanics sleeve 183 and biased the clamping arms 161 in a holding position. The intermediate sleeve 160 is axially fixed relative to the housing 180 and to the distal housing insert 181.

Furthermore, as shown in figure 7 and 8 the intermediate sleeve 160 includes on its levers 170 two radially inwards protruding actuation noses 178 adapted to abut locking arms 132 of an inserted syringe unit 102. Additionally, on a distal end each lever 170 includes a radially inwards protruding cap actuation nose 179 which are adapted to interact with the deflectable cap holding tongues 135 of the syringe unit 102. The trigger sleeve 150 includes lateral openings allowing the actuation nose 178 and the cap actuation nose 179 to extend through to reach the locking arms 132 and the tongues 135, respectively. The levers 170 comprise radially outside the actuation nose 178 counter cams 171 extending in a plane perpendicular to the longitudinal axis and comprising contact surfaces on a distal and on a proximal side as will be described in detail below. Correspondingly, the levers 170 comprise radially outside the cap actuation noses 179 counter cap cams 175 with contact surfaces. The contact surfaces of the counter cam 171 and of the counter cap cams 175 are adapted to interact with contact surfaces of control cams 151 and cap cams 155 of the trigger sleeve 160.

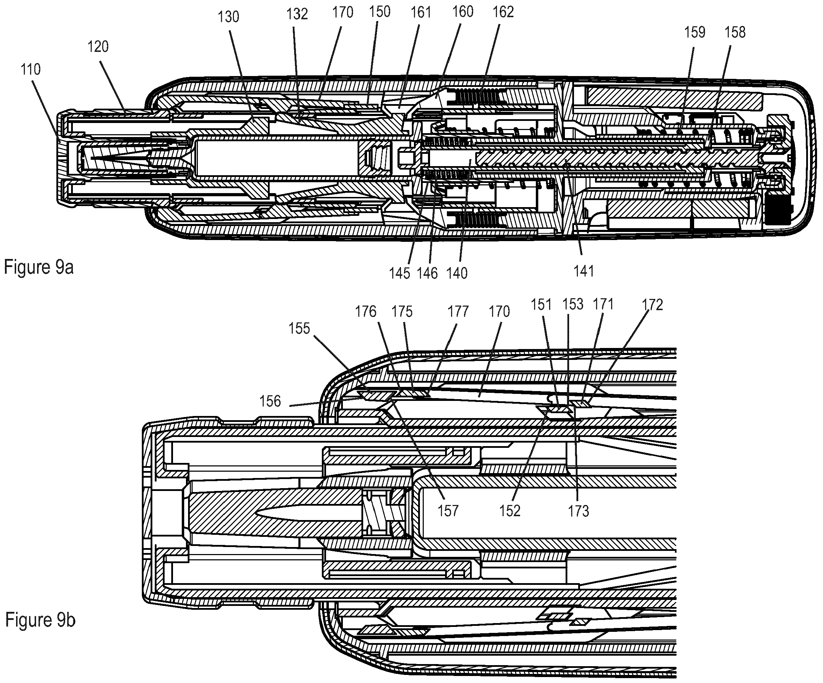

Figure 9a shows the autoinjector 100 in a unlocked state in a sectional view wherein the cut runs along the longitudinal axis. The syringe unit 102 is inserted into the drive unit 103 but not yet locked to the drive unit 103. Figure 9b shows the same state but the cut for the sectional view is offset from the longitudinal axis in order to show the control cams 151, cap cams 155 and counter cams 171 and counter cap cams 175.

As shown in figure 9b the trigger sleeve 150 control cams 151 are arranged on an outer portion oppositely to each other and extending in a plane perpendicular to the longitudinal axis. The control cams 151 are adapted to interact with the above mentioned counter cams 171 of the intermediate sleeve 160. Additionally, the cap cams 155 of trigger sleeve 150 are arranged on a distal end portion oppositely to each other and are adapted to interact with the corresponding counter cap cams 175 of the intermediate sleeve 160.

Further as shown in figure 9b the control cams 151 comprise on a distal side a first sloped contact surface 152 adapted to engage a second sloped contact surface 172 on a proximal side of the counter cam 171 of the intermediate sleeve 160. Furthermore, the control cam 151 comprises on a proximal side a third sloped contact surface 153 adapted to engage a fourth sloped contact surface 173 on a distal side of the counter cam 171. As shown in figure 9b all sloped contact surfaces 152, 153, 172, 173 have the same angle to the longitudinal axis.

Similarly, the cap cams 155 include a distal surface 156 and a proximal surface 157 and the counter cap cams 175 of the intermediate sleeve 160 include a counter distal surface 176 and a counter proximal surface 177. As it can be seen in figure 9b the distal and proximal surfaces of the cams 155, 175 are oppositely angled such that the cap cams 155 and counter cap cams 175 are shaped as a truncated pyramid in a plane parallel to the longitudinal axis.

The function of the second embodiment autoinjector 100 will be descripted in the following.

In a first step, the user inserts a proximal portion of the syringe unit 102 into the housing of the drive unit 103. Upon contact with the syringe unit ledges 133 the clamping arms 161 deflect radially and snap onto the proximal ledges 133 of the syringe holder 130 as described in the first embodiment. The syringe connector 147 biased by the syringe connector spring 148 abuts a proximal rim portion or flange of the syringe 15 and thus ensures that the syringe is pressed against a bearing surface inside the syringe holder 130.

The position sensor detects that the syringe unit 102 has been inserted and provides a corresponding signal to the controller. Subsequently, after a successful medication verification as described above with respect to the first embodiment the controller drives the electric motor 191 to automatically retract the trigger sleeve 150 via trigger sleeve connector 158 in a proximal direction.

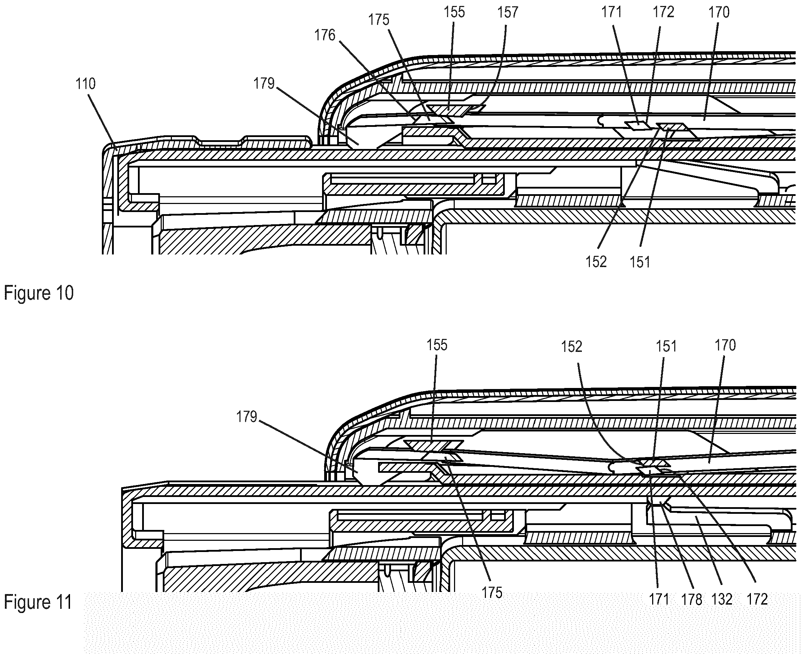

That means the trigger sleeve 150 is axially moved relative to the intermediate sleeve 160 which does not move. Therefore, the control cams 151 and the cap cams 155 of the trigger sleeve 150 move from an initial distal position as shown in figure 9b to a proximal position as shown in figure 10. During the proximal movement of the trigger sleeve 150 the control cams 151 thus pass radially inwards the counter cams 171 of the intermediate member 160. The cap cams 155, however, come into contact with their proximal sloped surface 157 with the distal sloped surface 176 of the counter cap cam 175 during trigger sleeve 150 movement relative to the intermediate sleeve 160.

Due to the movement of the trigger sleeve 150 the cap cam proximal sloped surface 157 slide along the counter cap cam distal surface 176 and thereby forcing the levers 170 of intermediate member 160 to deflect radially inwards which moves the cap actuation noses 179 radially inwards too. As the trigger sleeve 150 is further shifted proximally the cap cams 155 of the trigger sleeve passes above the counter cap cam 175 and thus keeps the levers 170 in a radially inwards deflected position as shown in figure 10 and 11.

In case the medication verification is not successful (e.g. an incorrect syringe unit was inserted) the controller drives the motor to move the trigger sleeve 150 in distal direction to release the syringe unit 102 from the drive unit 103 as described below with respect to the last step after an injection.

As the cap actuation noses 179 are now moved radially inwards the noses 179 press onto the protrusion 136 of the cap holding tongues 135 and thus deflect the holding tongues 135 radially inwards. This releases the ledges 137 from the cap 110 and thus the snap-fit connection between the tongues 135 and the cap 110 is released and the user can remove the cap 110 from the cover sleeve 120.

Subsequently, a cover sleeve lock is unlocked and the syringe unit 102 is locked to the drive unit 103. That is, the controller drives the motor 191 in dispensing direction causing the plunger rod 140 to move in distal direction. As the trigger sleeve connector 158 is pressed onto a proximal end of the plunger rod 140 by the trigger sleeve spring 159 (or alternatively the trigger sleeve connector is fixedly connected to the plunger rod) the trigger sleeve 150 is moved via trigger sleeve connector 158 in distal direction too.

This brings the first contact surfaces 152 of the control cams 151 into contact with the second contact surfaces 172 of the counter cams 171 of the intermediate member 160. As the first and second contact surfaces 152, 172 are sloped the two surfaces slide along each other and as the trigger sleeve 150 is axially guided and cannot move in radial direction the control cams 151 press the counter cams 171 radially inwards such that control cams 151 pass radially outwards the counter cams 171 (figure 11a). This bents or deflects a middle portion of the levers 170 of the intermediate sleeve 160 radially inwards and thereby moves the actuation noses 178 radially inwards as shown in figure 11a and 11b.

As a consequence, the actuation noses 178 press on the deflectable locking arms 132 of the syringe holder 130 thereby move the locking arms 132 radially inwards, see figure 11b. This in turn moves the free end of the locking arms 132 out of a locking position and the arms 132 no longer contact the stop surface of the cover sleeve 120. The cover sleeve 120 is thus unlocked and is able to move proximally relative to the syringe holder 130 and relative to the housing 180.

At the same time and in a manner analogous as in the first embodiment the radially inwards deflected actuation noses 178 abut a distal stop surface (not shown) of the syringe holder 130 and thereby lock the syringe holder 130 relative to the trigger sleeve 150 such that a movement of the trigger sleeve 150 is transferred to the syringe unit 2. Thus, in this state syringe unit 102 is locked inside the drive unit 3.

During the above descripted the distal movement of the trigger sleeve 150 the cap cams 155 are moved relative to the counter cap cams 175 of the intermediate sleeve 150. However, the cap cams 155 press still the counter cap cams 175 radially inwards and thus keep the cap actuation noses 179 in the radially inwards position as depicted in figure 11.

The controller displays a message on the display or activates LEDs to inform the user that the autoinjector is ready for and injection.

Figure 11b shows the above descripted state of the autoinjector 100 where the cap 110 is released and the cover sleeve 120 is unlocked and the syringe unit is locked by the radially inwards moved actuation noses 178 control.

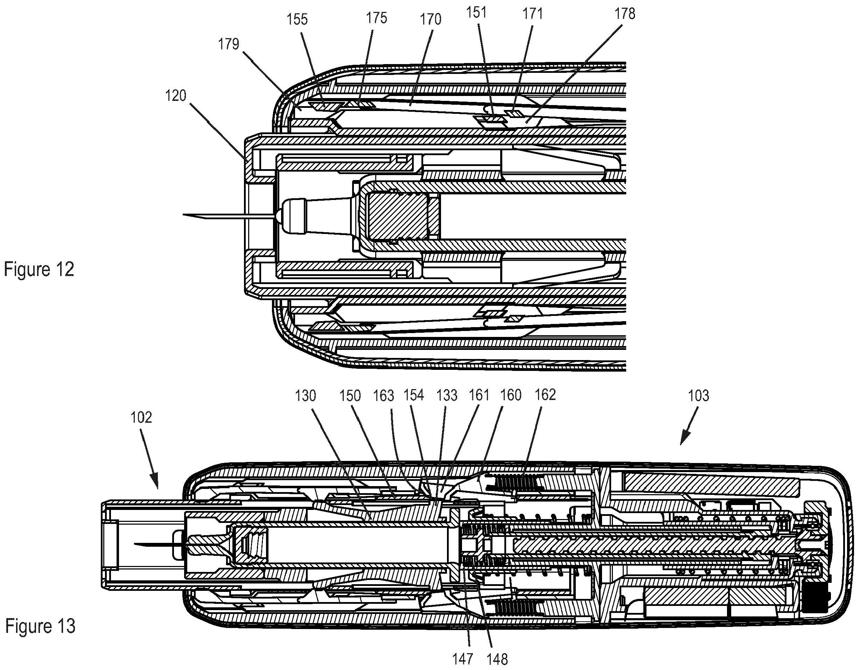

As described with respect to the first embodiment the injection is started by a push on skin release. Figure 12 depicts the state where the cover sleeve 120 is moved proximally out of the covering position if the user presses the autoinjector 100 onto the skin. The cover sleeve 120 moves the cover sleeve connector 145 proximally and thereby compressing the cover sleeve spring 146. In a proximal end position the a TMR position sensor detects via detection pin the proximal position of the retracted cover sleeve connector 145 and provides a start signal to the controller. The latter starts the injection by driving the motor 191 in dispensing direction.

The motor 191 rotates the threaded rod 141 and moves the non-rotating plunger rod 140 in distal direction and thus moves the trigger sleeve 150 via trigger sleeve connector 158 in distal direction. That means the trigger sleeve 150 with its control cams 151 and cap cams 155 is moved relative to the intermediate sleeve 160. The control cams 151 and cap cams 155 are moved from the position shown in figure 11a to the position shown in figure 12 wherein the control cams 151 and cap cams 155 pass and release the counter cams 171, 175 of the intermediate sleeve 160 and thus allow the intermediate sleeve levers 170 to deflect back into their initial non-deflected state. Thereby the actuation noses 178 and cap actuation noses 179 are move radially back outwards into their initial state. That would in turn return the syringe holder locking arms 132 towards their locking position. However, as the cover sleeve 120 is in a proximal retracted position the locking arms 132 cannot move completely radially outwards.

The motor 191 further drives the plunger rod 140 in distal dispensing direction and the plunger rod 140 moves the piston 16 inside the syringe 15 distally to dispense the liquid drug until the drug is completely dispensed. Subsequently, the controller displays a holding time counter as described with respect to the first embodiment.

When the user removes the autoinjector 100 from the injection site the cover sleeve 120 can move back into the covering position by a force of the compressed the cover sleeve spring 146. As the levers 170 of the intermediate sleeve 160 and thus the actuation nose 178 and the cap actuation nose 179 are in the retracted state the locking arms 132 of the syringe holder 130 can move back radially outwards once the cover sleeve 120 is moved back distally in its covering position. The cover sleeve 120 is therefore locked again by the locking arms 132 and thus is prevented from a movement in proximal direction.

The distal movement of the cover sleeve 120 moves the cover sleeve connector 145 too and the position sensor detects the movement via detection pin. Upon confirmation of the user via button the controller releases the lock of the syringe unit 102. For this purpose, the controller rotates the motor 191 in reverse direction to move the trigger sleeve 150 in a proximal end position. The proximal movement of the trigger sleeve 150 brings a proximal (third) contact surfaces 153 of the control cams 151 into contact with distal (fourth) contact surfaces 173 of the counter cams 171. Due to the sloped angle the counter cams 171 are moved radially outwards and thus move the levers 170 into their initial position (if the levers 170 are not already fully deflected back in the initial position). The control cams 151 and the cap cams 155 then pass the counter cams 171 and counter cap cams 175 respectively to a proximal position such that the cams of the trigger sleeve 150 do not contact the counter cams of the intermediate sleeve 160 anymore and thus levers 170 are not deflected any more.

If the trigger sleeve 150 is moved towards its proximal end position a proximal end wall 154 of the trigger sleeve 150 abuts a sloped release surface 163 of each clamping arm 161. If the trigger sleeve 150 is further moved in its proximal end position the end wall 154 presses the clamping arms 161 radially outwards and thus compressing the arm springs 162 as shown in figure 13. This releases the clamping arms from the ledges 133 and thus the syringe unit 102 is released from the drive unit 103. That means the compressed syringe connector spring 148 can relax thereby moving by the syringe connector 147 the entire unlocked syringe unit 102 in distal direction. The user can then remove the syringe unit 102 out of the drive unit housing 180. The controller drives the motor to move the trigger sleeve 150 distally back in its initial position. The autoinjector 100 is now ready to be loaded with a new syringe unit 102.

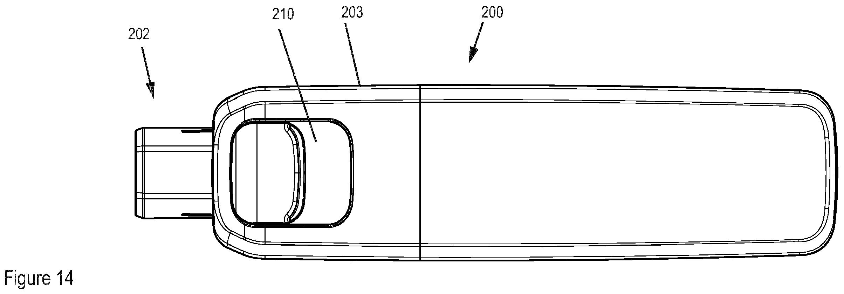

Figure 14 depicts a third embodiment with an autoinjector 200 according to the invention. In contrast to the second embodiment the drive unit 203 of the autoinjector 200 includes a slider 210 that can be shifted along the longitudinal axis by the user in order to move the syringe unit 202 out of the drive unit after an injection. Hence, in contrast to the above descripted last step (with respect to figure 13) the clamping arms are not deflected by a trigger sleeve movement but by a manual movement of the syringe unit by the user. All other features of the third embodiment are identical the above descripted second embodiment.

While the invention has been described in detail in the drawings and foregoing description, such description is to be considered illustrative or exemplary and not restrictive. Variations to the disclosed embodiments can be understood and effected by those skilled in the art and practising the claimed invention, from a study of the drawings, the disclosure, and the appended claims.

**LIST OF DESIGNATIONS**

| | | | | | |
|---|---|---|---|---|---|
| 1 | autoinjector | 80 | housing | 160 | intermediate sleeve |
| 2 | syringe unit | 81 | distal housing insert | 161 | clamping arms |
| 3 | drive unit | 82 | contact surface | 162 | spring |
| 4 | button | 83 | mechanics sleeve | 163 | release surface |
| | | 84 | wall | | |
| 10 | cap | 85 | proximal housing | 170 | lever |
| 11 | deflectable tongues | 86 | insert | 171 | counter cam |
| 12 | holder | | protrusion | 172 | second contact surface |
| 15 | prefilled syringe | 91 | electric motor | 173 | fourth contact surface |
| 16 | piston | 92 | | 175 | counter cap cam |
| 17 | rigid needle shield | 93 | pinion | 176 | counter distal surface |
| 18 | injection needle | 94 | gear battery | 177 178 179 | counter prox. surface actuation nose actuation nose |
| 20 | cover sleeve | 100 | | | cap |
| 21 | first opening | 102 | autoinjector unit | 180 | housing |
| 22 | second opening | 103 | syringe drive unit | 181 | distal housing insert |
| 23 | indentation | | | 183 | mechanics sleeve |
| 25 | label | 110 | | | |
| 26 | RFID tag | 120 | cap sleeve | 191 | motor |
| 27 | release surface | 130 132 | cover syringe holder | 200 | autoinjector |
| 30 | syringe holder | 133 | locking arms | 202 | syringe unit |
| 31 | longitudinal rail | 135 | ledges | 203 | drive unit |
| 32 | locking arms | 136 | cap holding tongues | 210 | slider |
| 33 | ledge | 137 | protrusion | | |
| 34 | bearing surface | 140 | ledge rod | | |
| 40 | plunger rod | 141 | plunger threaded rod | | |
| 41 | threaded rod | 145 | sleeve connector | | |
| 45 | cover sleeve connector | 146 | cover sleeve | | |
| 46 | cover sleeve spring | 147 | cover spring connector | | |
| 47 | syringe connector | 148 | syringe connector | | |
| 48 | syringe connector spring | | syringe spring | | |
| 49 | flange | 150 | trigger sleeve | | |
| 50 | trigger sleeve | 151 | control cam | | |
| 51 | trigger sleeve connector | 152 | first contact surface | | |
| 52 | ledge | 153 | third contact surface | | |
| 53 | trigger sleeve spring | 154 | end wall | | |
| 54 | trigger arm | 155 | camcap | | |
| 55 | arm sloped contact surface | 156 | cap distal surface | | |
| 56 | free end | 157 | cap prox. surface | | |
| | | 158 | trigger sleeve connector | | |
| 60 | gripper sleeve | 159 | sleeve trigger spring | | |
| 61 | clamping arms | | | | |
| 62 | clamp | | | | |
| 64 | gripper sleeve spring | | | | |
| 65 | counter release surface | | | | |

## Claims

1. A safety mechanism for a drug delivery device (1) for dispensing a liquid drug from a reservoir (15) through a needle (18), the safety mechanism comprising
- a housing (80) defining a longitudinal axis;
- a needle cover (20) movable along the longitudinal axis between a needle covering position, in which the needle (18) is covered and a retracted position, in which the needle (18) is exposed;
- a movable lock element (32) that can be in a locking position, in which the lock element (32) prevents the needle cover (20) from being moved out of the needle covering position;
**characterized in that**
the mechanism further includes a trigger member (50) movable along the longitudinal axis relative to the housing (80)
wherein a movement of the trigger member (50) along the longitudinal axis causes the lock element (32) to move out of the locking position thereby allowing the needle cover (20) to be moved out of the needle covering position.

2. Safety mechanism according to claim 1, wherein the safety mechanism includes a counter element (81, 171), and wherein a first guiding surface (82, 172) of the trigger member or the counter element causes the trigger member (50) or the counter element (81, 171) to move the lock element (32) out of the locking position if the trigger member (50) is moved along the longitudinal axis.

3. Safety mechanism according to claim 1 or 2, wherein the lock element (32) is a deflectable arm, wherein in the locking position a free end of the arm engages a stop surface of the needle cover (20).

4. Safety mechanism according to any of claims 1 to 3, wherein a movement of the trigger member (50) in a first direction along the longitudinal axis causes the lock element (32) to move radially out of the locking position and a movement of the trigger member (50) in a second, counter direction along the longitudinal axis enables the lock element (32) to move radially into the locking position.

5. Safety mechanism according to any of claims 2 to 4, wherein the trigger member (150) includes the first guiding surface and wherein the safety mechanism further includes an intermediate member (160) comprising the counter element (171) with a second guiding surface (172),
wherein a movement of the trigger member (150) along the longitudinal axis causes the first guiding surface (152) to interact with the second guiding surface (172) thereby moving a portion of the intermediate member (160) in a first radial direction or in an inclined direction which causes the lock element (132) to move out of the locking position.

6. Safety mechanism according to claim 5, wherein the trigger member (150) includes further a third guiding surface (153) and wherein the intermediate member (160) comprises a fourth guiding surface (173), wherein a movement of the trigger member (150) in a counter second longitudinal direction causes the third guiding surface (153) to interact with the fourth guiding surface (173) thereby moving a portion of the intermediate member (160) in a second radial direction allowing the lock element (132) to move into the locking position.

7. Safety mechanism according to claim 5 or 6, wherein the intermediate member (160) includes a radially protruding actuation element (178) adapted to interact with the lock element (132).

8. Safety mechanism according to claim 2 to 7, wherein the trigger member (150) includes a control element (151), wherein the first and third guiding surface (152, 153) are arranged each on an opposite sides of the control element (151) in a longitudinal direction.

9. Safety mechanism according to any of claims 1 to 8, comprising a cap (110) releasably attached at a distal end of the needle cover (120) in an initial state, wherein a deflectable element (135) of the needle cover or of the cap holds the cap (110) on the needle cover (120) in a non-deflected state, wherein a cap actuation element (179) can be actuated by the trigger member (150) and wherein a movement of the trigger member (150) in the longitudinal direction causes a radial movement of the cap actuation element (179) which causes a change of the deflectable element (135) to a deflected state thereby releasing the cap (110) from the needle cover (120).

10. Safety mechanism according to any of claims 2 to 4, wherein the housing (80) includes the counter element (81) wherein the movement of the trigger member (50) along the longitudinal axis causes the first guiding surface (55) to interact with the housing (80) thereby moving a portion of the trigger member (50) in a first radial direction or in a direction inclined with respect to the longitudinal axis thereby engaging and moving the lock element (32) out of the locking position.

11. Safety mechanism according to any of claims 2 to 4 or claim 10, comprising a cap (10) releasably attached at a distal end of the needle cover (20) in a shipping state, wherein a deflectable element (11) of the cap or of the needle cover holds the cap (10) on the needle cover (10), wherein a movement of the needle cover (20) out of the covering position causes the deflectable element (11) to change to a deflected state and thereby releasing the cap (10) from the needle cover (20).

12. An injection device (1) for dispensing the liquid drug from the reservoir (15), the injection device (1) comprising a plunger rod (40) and an automatic drive (91) for driving the plunger rod, wherein the device (1) further includes the safety mechanism according to any of claims 1 to 11.

13. Injection device according to claim 12, wherein the automatic drive (91) is adapted to rotate a drive member (41) which is operatively coupled to the plunger rod (40) and the trigger member (50), wherein rotating the drive member (41) in a first rotational direction causes the plunger rod (40) and the trigger member (50) to be moved in the first direction along the longitudinal axis and rotating the drive member (41) in a second, counter rotational direction causes the plunger rod (40) and the trigger member (50) to be moved in the second direction along the longitudinal axis.

14. Injection device according to claim 12 or 13 comprising a reservoir unit (2) and a drive unit (3), wherein the reservoir unit (2) comprises the reservoir (15) for the liquid drug, the needle cover (20) and the lock element (32) and wherein the drive unit (3) includes the housing (80), the automatic drive (91), the plunger rod (40) and the trigger member (50).

15. The invention further refers to a method for a safety mechanism for an injection device (1) for dispensing a liquid drug from a reservoir (15) through a needle (18). The method includes the steps of
a. Providing an injection device (1) with a housing (80) defining a longitudinal axis;
b. Moving a trigger member (50) inside the housing (80) along the longitudinal direction
c. Moving a lock element (32), by the trigger member (50) as the trigger member (50) is operatively coupled to the lock element (32), out of a locking position in which the lock element (32) prevents the needle cover from being moved out of a needle covering position,
d. Releasing, by the movement of the lock element (32), the needle cover (20) from the covering position and moving the needle cover out of the needle cover position.
